# EUROPEAN PATENT APPLICATION

(11) **EP 2 127 540 A1**
(43) Date of publication of application: **02.12.2009**
(21) Application number: 08704064.8
(22) Date of filing: 29.01.2008
(51) Int. Cl.: A23L 1/30, A23K 1/16, A61K 35/20, A61K 38/00, A61P 17/00

(54) **SKIN-BEAUTIFYING AGENT**

(30) Priority: 30.01.2007 JP 2007019448
(71) Applicant: Snow Brand Milk Products Co., Ltd., Sapporo-shi Hokkaido 065-0043 (JP)
(72) Inventor: KATO, Ken, Kawagoe-shi Saitama 350-1165 (JP); HARUTA, Yuko, Kawagoe-shi Saitama 350-1165 (JP); WATANABE, Tatsuya, Kawagoe-shi Saitama 350-1165 (JP); UENO, Hiroshi, Kawagoe-shi Saitama 350-1165 (JP); UEDA, Noriko, Kawagoe-shi Saitama 350-1165 (JP); YOSHIOKA, Toshimitsu, Kawagoe-shi Saitama 350-1165 (JP)
(74) Representative: Westendorp | Sommer
(86) International application number: PCT/JP2008/051257
(87) International publication number: WO 2008/093657

(57) **Abstract**

The present invention aims to provide a skin-beautifying agent, food, beverage, or feed for skin-beautification which has cosmetic effects such as moisturizing and beautifying the skin as well as preventing skin roughness and wrinkles by oral intake. The present invention further aims to provide a skin-beautifying agent containing a milk-derived phospholipid as an effective ingredient, and to provide food, beverage, or feed for skin-beautification containing the skin-beautifying agent. The milk-derived phospholipid is preferably a composition prepared from milk or milk material containing 40 to 70% by weight of lipid in a total solid content and containing 30% or more by weight of milk-derived phospholipid.

## Description

### Technical Field

The present invention relates to a skin-beautifying agent containing milk-derived phospholipid as an effective ingredient, which has cosmetic effects such as moisturizing and beautifying the skin as well as preventing skin roughness and wrinkles by oral intake. The present invention further relates to food, beverage, or feed for skin-beautification containing the skin-beautifying agent.

### Background Art

The skin serves as an interface between a living body and an external environment. The skin has a barrier function of preventing water loss from a body and blocking invasion of biologically harmful substances from the external environment such as microorganism and an allergen. Intercellular lipid of the stratum corneum, in which a major component is ceramide, sebum, and the like play such a function. The stratum corneum needs to contain 10 to 20% of water to function normally and maintain a healthy condition, and softness and elasticity of the skin are maintained by water retained in the stratum corneum owing to the skin barrier function.

Decrease in water in the stratum corneum causes loss of softness and hardening of the skin, which becomes a causative factor for cracking and the like. Water content in the stratum corneum significantly decreases in a so-called rough-textured skin, in which a skin pattern formed by raised skin ridges and fine skin grooves has disappeared or been vaguely defined. The rough-textured skin not only poses a cosmetic problem such that it gives poor appearance but it has a pathological significance in that it is a preliminary stage of a dermatologic disease. Also, improving the rough-textured condition of the skin changes dried skin surface to silky, smooth condition, leading to an improvement in fine wrinkles.

It is known that water loss from the skin is severer in the stratum corneum having a decreased skin barrier function compared to the stratum corneum in a healthy condition, where an increase is noted in Trans Epidermal Water Loss (TEWL). TEWL is regarded as an index of the skin barrier function based on its close association with the barrier function and moisturizing function of the stratum corneum.

Accordingly, it is possible to bring the skin to a healthy condition, or, in other words, a beautified condition, by increasing the water content of the skin or decreasing TEWL, or suppressing an increase in TEWL.

Furthermore, a problem of deterioration of the skin condition in animals, especially pets, due to effects of an allergy and the like has been pointed out in recent years. A healthy condition of the skin can be achieved by improving the deteriorated condition with skin moisturization and protection.

Meanwhile ceramide is one of components of the human skin and has effects of moisturizing and protecting the skin as well as effects of preventing and improving the skin roughness. As a cosmetic product using ceramide, a skin cosmetic preparation containing a ceramide such as ceramide, glucosylceramide, and galactosylceramide and diisopropylamine dichloroacetate or γ-aminobutyric acid is known (Patent Document 1). However, drawbacks were pointed out such that ceramide replenished to the skin was not absorbed and could not reach inside the skin due to interference by epidermal lipid, or components of cosmetic products and other products other than ceramide caused rash and inflammation. Furthermore, health food containing a ceramide composed of sphingosine, fatty acid, and sugar as an effective ingredient is known (Patent Document 2). As a raw material for the ceramide composed of sphingosine, fatty acid, and sugar, a product derived from konjac root, rice, wheat, and corn is on the market. However, a large volume of the raw material needs to be ingested to achieve an expected effect by oral intake because the above-mentioned raw materials contain little ceramide. Furthermore, the prices of the above-mentioned raw materials are expensive. For these reasons, the aforementioned health food has not been truly satisfactory.

Sphingomyelin accounts for about 30% of phospholipid in cow milk and has a structure in which phosphocholine is bound to a ceramide backbone composed of sphingosine and fatty acid. It is known to be largely distributed in the brain and the nerve tissue (Non-Patent Document 1). It is also known that food such as yolk contains a tiny amount of sphingomyelin. It is reported that sphingomyelin is taken up in a blood vessel via the small intestine when orally ingested (Non-Patent Document 2). It is known that sphingomyelin in the stratum granulosum is hydrolyzed by an action of sphingomyelinase and then supplied as ceramide in the stratum corneum (Non-Patent Document 3). In addition, it is reported that sphingomyelin exerts superior cosmetic effects such as moisturizing and protecting the skin, preventing and improving the skin roughness as well as preventing wrinkles by ingestion of a smaller amount of a raw material containing sphingomyelin than that containing a ceramide (Patent Document 3). However, generally an organic solvent such as ethanol needs to be used to upgrade a purity of sphingomyelin, which raises a safety issue. In addition, a skin-beautifying effect obtained by oral intake of the raw material containing sphingomyelin has not been necessarily satisfactory. In view of the above, development of a material which exerts a superior skin-beautifying effect with higher safety is awaited.
Patent Document 1: Japanese Patent Laid-Open No. 1-22810
Patent Document 2: Japanese Patent Laid-Open No. 11-113530
Patent Document 3: Japanese Patent Laid-Open No. 2005-281257
Non-Patent Document 1: Harper's Biochemistry, the 24th edition, pp. 162, 1997
Non-Patent Document 2: Schmelz et al., J. Nutr., 124, pp. 702-712, 1994
Non-Patent Document 3: Yoshikazu Uchida et al., Biochemistry, Vol. 73, 4, pp. 269-272, 2001

### Disclosure of the Invention

### Problems to be Solved by the Invention

The present invention aims to provide a highly safe skin-beautifying agent which exerts excellent cosmetic effects such as moisturizing and protecting the skin, preventing and improving the skin roughness as well as preventing wrinkles by oral intake. The present invention further aims to provide food, beverage, or feed for skin-beautification containing a skin-beautifying agent which exerts excellent cosmetic effects such as moisturizing and protecting the skin, preventing and improving the skin roughness as well as preventing wrinkles by oral intake.

### Means for Solving the Problems

In view of the foregoing problems, the present inventors conducted extensive and thorough research about an ingredient which exerts superior cosmetic effects such as moisturizing and protecting the skin, preventing and improving the skin roughness as well as preventing wrinkles, and which is obtained from a highly safe raw material. As a result, the present inventors found out that cosmetic effects such as moisturizing and protecting the skin, preventing and improving the skin roughness as well as preventing wrinkles can be obtained by orally ingesting milk-derived phospholipid, and completed the present invention by providing milk-derived phospholipid as an effective ingredient.

### Advantages of the Invention

A skin-beautifying agent containing milk-derived phospholipid as an effective ingredient and food, beverage, or feed for skin-beautification containing the skin-beautifying agent can be provided according to the present invention. The skin-beautifying agent of the present invention is safe because the effective ingredient is derived from food and no organic solvent is used in its preparation process, and it can provide excellent cosmetic effects such as moisturizing and protecting the skin, preventing and improving the skin roughness as well as preventing wrinkles.

### Best Mode for Carrying Out the Invention

The present invention is characterized by provision of milk-derived phospholipid as an effective ingredient. The milk-derived phospholipid used in the present invention can be ones prepared from milk of mammals such as cow, goat, sheep, and human. The milk-derived phospholipid used in the present invention further can be ones prepared from milk materials such as butter serum and butter milk, which are prepared from milk of mammals as described above. In fact, as a raw material of the milk-derived phospholipid derived from cow milk, ones containing the milk-derived phospholipid at a concentration of as high as 30% or more are on the market at a low price; therefore, these ones can be used in the present invention.

The aforementioned milk-derived phospholipid can be provided as is as the skin-beautifying agent of the present invention, while it is possible to provide the skin-beautifying agent of the present invention as a nutritional composition in which the milk-derived phospholipid and raw ingredients and the like which are normally used for pharmaceutical products, food, beverage, and feed such as sugars, lipid, protein, vitamins, minerals, and flavor are mixed. It is further possible to prepare the skin-beautifying agent of the present invention in a form of powder, granule, tablet, capsule, and drink formulations, following a conventional method. It is further possible to provide the milk-derived phospholipid with other ingredients which exert a cosmetic effect, for example, collagen, vitamin C, iron, and the like, which promote a production of collagen in the skin.

Regarding an effective amount of the skin-beautifying agent of the present invention, it was found by the animal test using hairless mice as will be described below that a water content in the skin was increased and TEWL was decreased by oral intake of 6 mg or more, preferably 15 mg or more of milk-derived phospholipid per kilogram of body weight of the mouse. Accordingly, cosmetic effects such as moisturizing and protecting the skin, preventing and improving the skin roughness as well as preventing wrinkles can be expected by ingestion of 6 mg or more, preferably 15 mg or more of the milk-derived phospholipid per adult per day. Based on the above, the skin-beautifying agent of the present invention can be provided so that the necessary amount of the milk-derived phospholipid therein is ensured.

The food and the beverage for skin-beautification according to the present invention can be provided by incorporating the skin-beautifying agent of the present invention in ordinary food and beverage, for example, yogurt, milk beverage, wafer, desserts, and the like. In the food and the beverage for skin-beautification, it is preferable to incorporate the skin-beautifying agent therein so that the content of the milk-derived phospholipid is 0.5 to 2000 mg per 100 g of the food and the beverage in order to achieve ingestion of 6 mg or more of the milk-derived phospholipid per adult per day, although the amount varies depending on a form of the food and the beverage.

The feed for skin-beautification according to the present invention can be provided by incorporating the skin-beautifying agent of the present invention in ordinary feed, for example, feed for livestock, pet food, and the like. In the feed for skin-beautification, it is preferable to incorporate the skin-beautifying agent therein so that the content of the milk-derived phospholipid is 0.5 to 2000 mg per 100 g of the feed in order to achieve ingestion of 6 mg or more of the milk-derived phospholipid.

According to the present invention, no particular limitation is imposed on a method for incorporating the skin-beautifying agent. For example, in order to conduct addition and incorporation in a solution, the skin-beautifying agent is suspended or dissolved in deionized water and then mixed with stirring. The obtained mixture is prepared into forms such as pharmaceutical products, food, beverage, and feed for use. As for a condition of stirring and mixing, the skin-beautifying agent needs to be uniformly mixed, and it is possible to conduct stirring and mixing using an ultra-disperser, a TK homomixer, and the like. Further, the resulting solution of milk-derived phospholipid can be concentrated using a reverse osmosis (RO) membrane and the like or dried by freeze-drying and the like for use as needed in an aim to make application thereof into pharmaceutical products, food, beverage, or feed easier.

According to the present invention, a sterilization treatment normally used in a production of pharmaceutical products, food, beverage, and feed is feasible, and dry heat sterilization can be conducted when the present invention is provided in a form of powder. Accordingly, the pharmaceutical products, the food, the beverage, and the feed of the present invention containing milk-derived phospholipid can be produced in various forms such as liquid, gel, powder, and granule.

### Examples

The present invention is further described in detail hereinbelow with examples and test examples. However, these examples are provided for an illustrative purpose only and the present invention is by no means limited in any way by these examples.

### (Example 1)

A 25% solution of butter serum powder (SM2, manufactured by Corman S. A.) was prepared, to which 5 N hydrochloric acid was added to adjust pH to 4.5. The solution thus obtained was left to stand for one hour at 50°C to precipitate casein proteins. The resulting precipitates were filtered out using a filter press. The solution thus obtained was treated with an MF membrane having a pore size of 1.0 µm (manufactured by SCT) to give a concentrated liquid fraction. The concentrated liquid fraction thus obtained was freeze-dried, thereby a composition containing milk-derived phospholipid was obtained. The composition thus obtained contained 53% of lipid, 31% of phospholipid, 24% of protein, 15% of sugar, and 8% of ash in a total solid content, in which sphingomyelin accounted 20% of the phospholipid.

### (Example 2)

A 20% solution of butter milk powder (manufactured by Snow Brand Milk Products Co., Ltd.) was prepared, to which 2N hydrochloric acid was added to adjust pH to 4.5. The solution thus obtained was left to stand for 30 minutes at 45°C to precipitate casein proteins. The resulting precipitates were removed using a clarifier. The supernatant thus obtained was treated with an MF membrane having a pore size of 0.1 µm (manufactured by SCT) to give a concentrated liquid fraction. The concentrated liquid fraction thus obtained was freeze-dried, thereby a composition containing milk-derived phospholipid was obtained. The composition thus obtained contained 62% of lipid, 38% of phospholipid, 15% of protein, 18% of sugar, and 5% of ash per solid content, in which sphingomyelin accounted 20% of phospholipid.

### [Test Example 1]

### (Animal test)

A skin-beautifying effect of milk-derived phospholipid was evaluated using the compositions containing milk-derived phospholipid obtained in Examples 1 and 2. In experiments 13-week-old hairless mice (Hos: HR-1) were used. The mice were divided into 11 test groups, each group containing 8 mice, as follows; group A to which physiological saline was administered at 10 g per kilogram of mouse body weight; group B to which the composition containing milk-derived phospholipid obtained in Example 1 was administered at 6 mg in terms of milk-derived phospholipid per kilogram of mouse body weight; group C to which the composition containing milk-derived phospholipid obtained in Example 1 was administered at 15 mg in terms of milk-derived phospholipid per kilogram of mouse body weight; group D to which the composition containing milk-derived phospholipid obtained in Example 1 was administered at 30 mg in terms of milk-derived phospholipid per kilogram of mouse body weight; group E to which the composition containing milk-derived phospholipid obtained in Example 2 was administered at 6 mg in terms of milk-derived phospholipid per kilogram of mouse body weight; group F to which the composition containing milk-derived phospholipid obtained in Example 2 was administered at 15 mg in terms of milk-derived phospholipid per kilogram of mouse body weight; group G to which the composition containing milk-derived phospholipid obtained in Example 2 was administered at 30 mg in terms of milk-derived phospholipid per kilogram of mouse body weight; group H to which a fraction of the composition containing milk-derived phospholipid obtained in Example 1 from which sphingomyelin had been removed was administered at 6 mg in terms of phospholipid per kilogram of mouse body weight; group I to which a fraction of the composition containing milk-derived phospholipid obtained in Example 1 from which sphingomyelin had been removed was administered at 15 mg in terms of phospholipid per kilogram of mouse body weight; group J to which a fraction of the composition containing milk-derived phospholipid obtained in Example 1 from which sphingomyelin had been removed was administered at 30 mg in terms of phospholipid per kilogram of mouse body weight; group K to which sphingomyelin was administered at 3 mg per kilogram of mouse body weight to be provided as a positive control. The respective substances were orally administered by a sonde once daily and the mice were reared for three weeks. The compositions containing milk-derived phospholipid obtained in Examples 1 and 2, the fraction of the composition containing milk-derived phospholipid obtained in Example 1 from which sphingomyelin had been removed, and sphingomyelin were each suspended in 10 g of physiological saline and administered orally to groups B to K, respectively. A water content and TEWL of the skin of the tail and the base of the tail of the mice were measured at the initiation of the test and the completion of the test. Values obtained at the completion of the test were calculated by setting each value obtained at the initiation of the test as 100, and the obtained values were provided as a rate of increase. A water content of the skin and TEWL were measured with a Corneometer and a Tewameter manufactured by Courage + Khazaka electronic GmbH, respectively. The results are shown in Table 1.

**[Table 1]**

| | Amount of PL intake | Amount of SPM intake | Rate of water content increase | Rate of TEWL increase |
|---|---|---|---|---|
| | (mg/day) | (mg/day) | (%) | (%) |
| Group A | 0 | 0 | 78 | 102 |
| Group B | 6 | 1.2 | 124 | 86 |
| Group C | 15 | 3 | 148 | 82 |
| Group D | 30 | 6 | 152 | 79 |
| Group E | 6 | 1.2 | 125 | 85 |
| Group F | 15 | 3 | 150 | 81 |
| Group G | 30 | 6 | 154 | 78 |
| Group H | 6 | -- | 108 | 95 |
| Group I | 15 | -- | 116 | 89 |
| Group J | 30 | -- | 120 | 87 |
| Group K | -- | 3 | 124 | 86 |

| | | | | |
|---|---|---|---|---|
| PL: milk-derived phospholipid (sphingomyelin had been removed in Groups H to J) SPM: sphingomyelin | | | | |

As a result, the water content of the skin decreased in group A, while it increased to about 1.25 times as much as the original values in groups B and E, and to about 1.5 times as much as the original values in groups C, F, D, and G after three weeks of administration. On the other hand, the water content of the skin in group K, which was a positive control, to which sphingomyelin was administered solely, was 1.25 times as much as the water content of the skin of group A. TEWL changed little in group A, while it was decreased to about 0.85 times as much as the original values in groups B and E, and to about 0.8 times as much as the original values in groups C, F, D and G after three weeks of administration. On the other hand, the water content of the skin in group K, which was a positive control, to which sphingomyelin was administered solely, was 0.85 times as much as the water content of the skin of group A. The above results revealed that the water content of the skin was increased, while TEWL was decreased by oral intake of milk-derived phospholipid. Also, the above-described effect was higher when milk-derived phospholipid containing sphingomyelin was administered compared to the cases in which sphingomyelin was solely administered. Furthermore, the above-described effect was obtained when milk-derived phospholipid was administered at 6 mg or more per kilogram of mouse body weight, and a marked effect was obtained when milk-derived phospholipid was administered at 15 mg or more per kilogram of mouse body weight.

On the other hand, improvements were noted in the water content of the skin and TEWL in groups H, I, and J, to which the compositions containing milk-derived phospholipid from which sphingomyelin had been removed were administered; however, the effects thus obtained were lower than the effects in group K to which sphingomyelin was solely administered. Also, a comparative review of groups C, F, I, and K revealed that superior effects could be obtained when milk-derived phospholipid containing both sphingomyelin and phospholipid other than sphingomyelin was administered compared to the cases to which sphingomyelin and phospholipid other than sphingomyelin were each administered independently.

### (Example 3)

In 4950 g of deionized water, 50 g of the composition containing milk-derived phospholipid obtained in Example 1 was dissolved. The resulting mixture was heated to 50°C and mixed with stirring for 30 minutes at 6000 rpm using a TK homomixer (type: TK ROBO MICS, manufactured by Tokushu Kika Kogyo Co., Ltd.), thereby a solution of milk-derived phospholipid containing 310 mg of milk-derived phospholipid per 100 g of the solution was obtained. In 4.0 kg of the solution of the composition containing milk-derived phospholipid thus obtained, 5.0 kg of casein, 5.0 kg of soy protein, 1.0 kg of fish oil, 3.0 kg of perilla oil, 18.0 kg of dextrin, 6.0 kg of mineral mixture, 1.95 kg of vitamin mixture, 2.0 kg of emulsifier, 4.0 kg of stabilizer, and 0.05 kg of flavor were incorporated. The resulting mixture was packed in a 200 mL retort pouch and subjected to sterilization in a retort sterilizer (a class I pressure vessel, type: RCS-4CRTGN, manufactured by Hisaka Works, Ltd.) for 20 minutes at 121 °C, thereby 50 kg of liquid nutritional composition for skin-beautification containing the skin-beautifying agent of the present invention was produced. The liquid nutritional composition for skin-beautification thus obtained contained, per 100 g thereof, 24.8 mg of milk-derived phospholipid, which was an effective ingredient of the skin-beautifying agent.

### (Example 4)

In 700 g of deionized water, 10 g of the composition containing milk-derived phospholipid obtained in Example 1 was dissolved. The resulting mixture was heated to 50°C and mixed with stirring for 30 minutes at 9500 rpm using an ultra-disperser (type: ULTRA-TURRAX T-25, manufactured by IKA Japan Y.K.), thereby a solution was obtained. To the solution thus obtained, 40 g of sorbitol, 2 g of acidulant, 2 g of flavor, 5 g of pectin, 5 g of lactoserum protein concentrate, 1 g of calcium lactate, and 235 g of deionized water were added and mixed with stirring. The resulting mixture was packed in a 200 mL cheer pack and subjected to sterilization for 20 minutes at 85°C. The product thus obtained was sealed, thereby 5 packs (200 g each) of gel food for skin-beautification containing the skin-beautifying agent of the present invention were prepared. None of the gel food for skin-beautification thus obtained produced precipitation or the like, and no abnormality was found in flavor. The gel food for skin-beautification thus obtained contained, per 100 g thereof, 310 mg of milk-derived phospholipid, which was an effective ingredient of the skin-beautifying agent.

### (Example 5)

In 700 g of deionized water, 2 g of acidulant was dissolved and then 10 g of the composition containing milk-derived phospholipid obtained in Example 2 was dissolved. The resulting mixture was heated to 50°C and mixed with stirring for 30 minutes at 9500 rpm using an ultra-disperser (type: ULTRA-TURRAX T-25, manufactured by IKA Japan Y.K.), thereby a solution was obtained. To the solution thus obtained, 100 g of maltitol, 20 g of reduced starch syrup, 2 g of flavor, and 166 g of deionized water were added, and the resulting mixture was filled in a 100 mL glass bottle and subjected to sterilization for 15 minutes at 90°C. The product thus obtained was sealed, thereby 10 bottles (100 mL each) of beverage for skin-beautification containing the skin-beautifying agent of the present invention were prepared. None of the beverage for skin-beautification thus obtained produced precipitation or the like, and no abnormality was found in flavor. The beverage for skin-beautification thus obtained contained, per 100 g thereof, 380 mg of milk-derived phospholipid, which was an effective ingredient of the skin-beautifying agent.

### (Example 6)

In 98 kg of deionized water, 2 kg of the composition containing milk-derived phospholipid obtained in Example 2 was dissolved. The resulting mixture was heated to 50°C and mixed with stirring for 40 minutes at 3600 rpm using a TK homomixer (type: MARKII 160, manufactured by Tokushu Kika Kogyo Co., Ltd.), thereby a solution of milk-derived phospholipid containing 760 mg of milk-derived phospholipid per 100 g of the solution was obtained. In 10 kg of the solution of the composition containing milk-derived phospholipid thus obtained, 12 kg of soybean meal, 14 kg of nonfat dry milk, 4 kg of soy oil, 2 kg of corn oil, 23.2 kg of palm oil, 14 kg of corn starch, 9 kg of wheat flour, 2 kg of bran, 5 kg of vitamin mixture, 2.8 kg of cellulose, and 2 kg of mineral mixture were incorporated. The resulting mixture was subjected to sterilization for 4 minutes at 120°C, thereby 100 kg of dog feed for skin-beautification containing the skin-beautifying agent of the present invention was produced. The dog feed for skin-beautification thus obtained contained, per 100 g thereof, 76 mg of milk-derived phospholipid, which was an effective ingredient of the skin-beautifying agent.

### (Example 7)

Raw ingredients were mixed according to a composition shown in Table 2, and formed into a 1-g tablet following a conventional method to produce the skin-beautifying agent of the present invention. The skin-beautifying agent thus obtained contained, per gram thereof, 31 mg of milk-derived phospholipid, which was an effective ingredient of the skin-beautifying agent.

**[Table 2]**

| Hydrous crystalline glucose | 83.5% (wt%) |
|---|---|
| The composition containing milk-derived phospholipid (Example 1) | 10.0 |
| Mineral mixture | 5.0 |
| Sugar ester | 1.0 |
| Flavor | 0.5 |

### Industrial Applicability

The skin-beautifying agent of the present invention containing milk-derived phospholipid as an effective ingredient, as well as food, beverage, and feed containing the skin-beautifying agent have high applicability as they can be used for moisturizing and beautifying the skin as well as preventing skin roughness and wrinkles, and the like.

## Claims

1. A skin-beautifying agent comprising a milk-derived phospholipid as an effective ingredient.

2. The skin-beautifying agent according to Claim 1, wherein a composition comprising a milk-derived phospholipid prepared from milk or milk material is used as the milk-derived phospholipid, wherein the composition comprises 40 to 70% by weight of lipid in a total solid content and 30% or more by weight of milk-derived phospholipid in the total solid content.

3. The skin-beautifying agent according to Claim 2, wherein the composition comprising the milk-derived phospholipid is obtained by treating milk or milk material through a membrane having a pore size of 0.1 to 2.0 µm or a membrane having a molecular weight cut-off of 5 to 500 kDa.

4. The skin-beautifying agent according to Claim 2, wherein the composition comprising the milk-derived phospholipid is obtained by adding an acid to milk or milk material to adjust pH to 4.0 to 5.0 and removing casein protein as a precipitate, followed by treatment with a membrane having a pore size of 0.1 to 2.0 µm or a membrane having a molecular weight cut-off of 5 to 500 kDa.

5. The skin-beautifying agent according to any one of Claims 2 to 4, wherein the milk or the milk material is butter serum or butter milk.

6. A food or beverage for skin-beautification comprising the skin-beautifying agent according to any one of Claims 1 to 5.

7. A feed for skin-beautification comprising the skin-beautifying agent according to any of Claims 1 to 5.
